# EUROPEAN PATENT SPECIFICATION

(11) **EP 1 511 416 B1**
(45) Date of publication and mention of the grant of the patent: **13.08.2008**
(21) Application number: 03757506.5
(22) Date of filing: 10.06.2003
(51) Int. Cl.: A61B 1/24

(54) **COMPACT DIGITAL INTRAORAL CAMERA**
KOMPAKTE INTRAORALE DIGITALKAMERA
CAMERA INTRA-ORALE NUMERIQUE COMPACTE

(30) Priority: 10.06.2002 US 387465 P; 10.06.2003 US 458417
(43) Date of publication of application: 09.03.2005
(73) Proprietor: Gendex Corporation, Washington, DC 20006-1813 (US)
(72) Inventor: GREGORIO, John, Joseph, Chana, IL 61015 (US); JAMBOR, Steven, R., Chicago, IL 60618 (US); COOPER, David, Harvey, Carlsbad, CA 92009 (US)
(74) Representative: Hartz, Nikolai
(86) International application number: PCT/US2003/019738
(87) International publication number: WO 2003/103483

(56) References cited:
- WO-A-02/082820
- US-A1- 2001 055 368
- US-A1- 2002 067 407

## Description

### TECHNICAL FIELD

The present invention is a true digital output intraoral camera with a compact docking station.

### BACKGROUND OF THE INVENTION

Intraoral cameras such as The AcuCam Concept III from DENTSPLY International Inc., are known to be useful in the art. Real time images of the oral cavity can be displayed for purposes of diagnosis, treatment, patient education and the like.

It will be appreciated that the dental professional has a wide range of useful equipment with which patient treatments can be effected as known for example from US 2002/067 407, which relates to a docking station for managing o plurality of dental imaging subsystems. While this results in improved patient care, the professional must be cognizant of the physical space requirements of equipment and the office within which it is used.

A need exists therefore, for smaller and improved such equipment. According to the present invention, a camera system provides digital video output and has the ability to integrate into existing dental office furniture such as the dental chair /unit or the cabinetry in a space-saving manner.

### BRIEF DESCRIPTION OF THE DRAWING

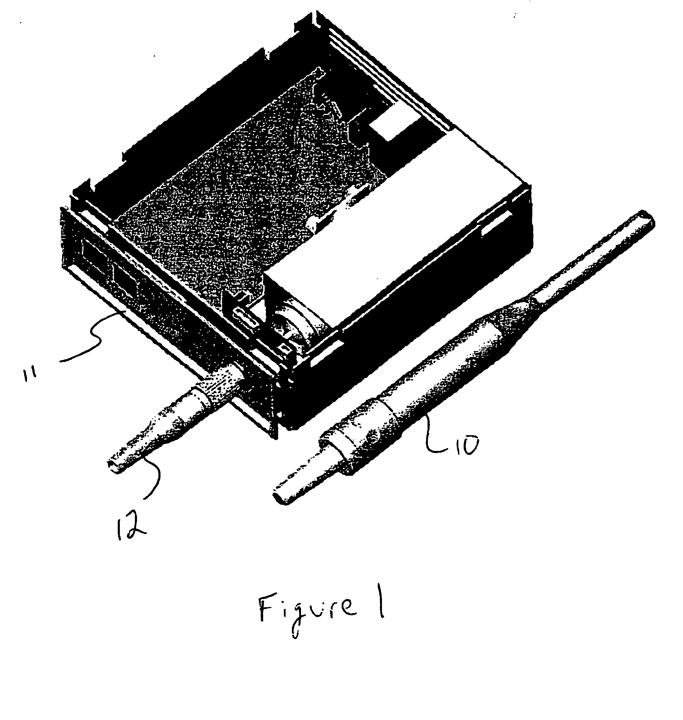

The drawing Figure 1 shows an intraoral camera system according to the concepts of the present invention.

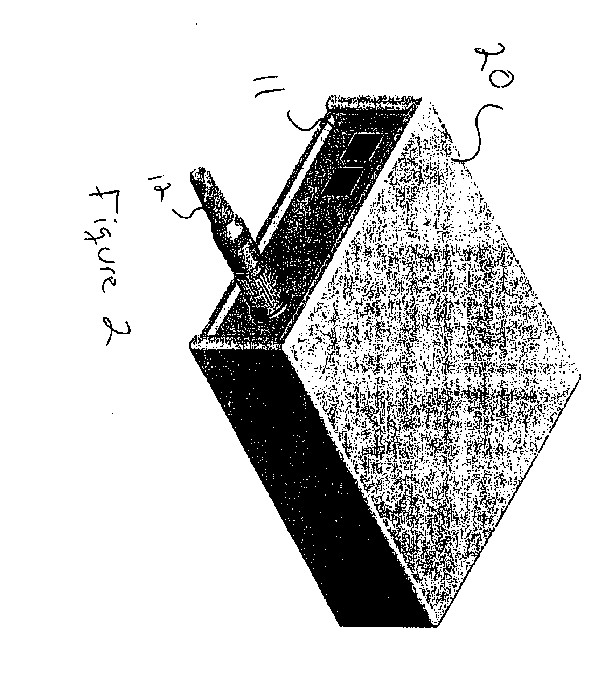

Figure 2 shows the docking station of the system of Figure 1 with a cover in place.

### PREFERRED EMBODIMENT FOR CARRYING OUT THE INVENTION

The present invention provides an intraoral camera 10 having a docking station 11 that is miniaturized in order to fit into smaller spaces in the dental operatory. It is preferably designed to fit under the delivery unit of a conventional dental chair. With the present invention, a dedicated footswitch is no longer necessary because images will be saved via the computer. However, the new docking station may have the connection and electronics to connect a conventional footswitch. According to an embodiment of the invention, control commands are transferred via a Firewire connection 12.

This product is a dental intraoral camera 10 with direct digital video output via Firewire 12 connection and protocol (IEEE 1394 standard). It employs a conventional intraoral camera handpiece 10, such as DENTSPLY's AcuCam Concept IV handpiece camera, or a variation thereof, with the new docking station 11. The docking station 11 is more compact than in the station's heretofore known in the art, and can be integrated with a dental unit (chair) or in a dental cabinet. The docking station 11 is preferably powered by a low-voltage external supply. It includes the electronics necessary to convert the handpiece S-Video to a Firewire digital output. This output interfaces to any Firewire equipped computer that complies with the IEEE 1394A standard.

As an example, a digital intraoral camera having a compact foorprint according to an embodiment of the invention, has the following general characteristics.

Compact footprint approximately 150 x 150 x 50 mm, or smaller.

Single S-Video input via a Lemo type 10-pin connector with fiberoptic ferrule.

Provides standard S-Video and uncompressed Firewire outputs.

The power supply physical size does not exceed approximately 100 X 100 X 50 and preferably has two cables one to the AC input and the other end to the Docking station.

Input power range from 90 to 265V, 50-60 Hz.

Provides an IEEE1394A Firewire output to interface to a standard Windows Computer using Windows OS via a 3-4.5 Meters Firewire cable.

Provides S-Video output to external devices.

A switch turn off the lamp and blank the video signal when the handpiece is holstered.

Ergonomic docking station designed for dental chair, mounting, wall mount, counter top, or flush mounting in a cabinet.

Size/shape compatible with most popular chair systems, e.g. Adec, or Royal and for mounting below a delivery system.

Footswitch connection for routing to a computer.

The chassis is preferably formed from 1-mm thick, zinc plated steel, with ventilation slots to provide the necessary forced air cooling of critical components. The ventilation slots conform to the IEC-60601-1 requirements to prevent a standard probe from reaching electrical components. The Docking Station provides a holster to hold the handpiece, which may be attached to the chassis, or located remotely. The holster will have a built-in light sensor to implement automatic lamp and camera activation when the instrument is removed from the holster. The chassis top cover snaps into slots in the chassis sides and provides an EMC environment. Brackets will be provided to enable flush mounting in cabinets.

The top cover 20 or bezel is preferably molded from UV stabilized Lexan, or equivalent material. The material should conform to UL-2601, FDA and IEC-60601-1 requirements. The top cover is applied in counter-top, chair-mount and wall-mount applications. The bezel is used when the Docking Station is flush mount in cabinet space. The top cover and bezel are designed to be aesthetically and ergonomically pleasing, with clean modem lines and suitable for a medical environment. They will be essentially smooth to permit easy cleaning, and free of ridges, or fluting, which could form a bacteria trap and will mate aesthetically and cleanly with a molded front connector panel, which will support the Lemo type receptacle and provide the necessary isolation.

The docking station components include a light source assembly; a Firewire/video board with patient connected circuit isolation; a power supply board with regulator and filter circuits that may be combined with the Firewire/video board; a Single, low noise 12 VDC fan with ball bearings; a 12VDC input connector, Firewire, S Video and footswitch output connectors; Lemo type receptacle connector board as conventionally used in the Concept IV docking station; a top cover; substantially total EMI shielding; and a 12VDC external power supply.

The present system uses a docking station having a light source, such as a conventional source. For example, on useful light source is that used with the Concept IV docking station, which has a 2,000 hour, 12 volt, 75W-halogen lamp, with a modified mount. The focusing system consists of a spherical reflector and a pair of aspherical focusing elements. All optical elements are mounted in such a way to avoid differential expansion problems.

The light source assembly is preferably mounted in an aluminum tube with an ID of approximately 25mm. The tube is cooled by a low noise, 12 VDC fan and is provided with a thermostatic circuit breaker to cut off the lamp current if the temperature rises above 167F in event of fan failure. The light source chassis mount is designed to force the entire flow of air through the fan to pass over the tube. Easy access is provided for lamp replacement by removing the top cover. The fan is powered from the 12VDC power input via suitable EMI filter components.

The docking station preferably includes a holster to support the camera head and cable when not in use. The lamp is turned off and the video signal is blanked when the camera is in the holster. The holster can either be mounted on the chassis, or remotely. Preferably, the holster will include a passive means to sense the presence of the handpiece, such as a magnet interacting with hall effect, or magneto-resistive element in the handpiece, or the like. Alternatively, a version is within the scope of the invention which utilizes a sensor assembly (Led/photo-transistor set) to detect the presence of the camera in the holster. The preferred modification provides a miniature 4-pin connector located in the hollow compartment of an otherwise conventional holster, such as that found in the Concept IV holster. This connects directly to the sensor assembly when the holster is chassis mounted, or via and extension cable in remote applications.

In the case of the magnetic holster, the handpiece sensor will be connected through the camera cable to the FirewireNideo board via an optical isolator. This would utilize the ATW wire, if available in the new HRDSP design (to be confirmed). In the case of the conventional and modified Concept IV holster, the sensor will be connected by a harness to the Firewire/Video board.

General functional performance characteristics of an embodiment of the present invention include:

The Firewire will provide 4.2.2 video to a computer.

Handpiece S-Video input, Firewire output with no compression,(DCAM standard).

Full performance, 480 TV lines (support) NTSC, or 525 TV lines PAL formats.

Provides footswitch control (this), which information is then passed (to the software in) Firewire to the computer.

Analog S-Video output connector interfacing to an S-Video peripherals.

### Supports isochronous transfers.

General operation of the hardware interface includes the following:

The system provides S-video isolation via video optical isolators.
Then the S-video signal is converted from analog S-video to 4:2:2 digital data and sent to the link layer controller chip.

The data is converted into packets IEEE1394 Serializes the video signal data and sends them to the Physical Layer output chip.

The Microprocessor will program the Video decoder and the Firewire chips.

The CPLD does the address mapping and other I/O functions.

The CPLD can be programmed via a JTAG port and the Microprocessor can be programmed via the RS232 port.

I²C support to control the handpiece programming.

Sensing of the removal of the handpiece.

1500V isolation of the patient connected circuits, including the S-Video input, the circuit to sense if the handpiece is plugged into the docking station, and whether the handpiece is holstered, if a magnetic sensor is used.

A Firewire chip set to provide an uncompressed isochronous Firewire video output.

A footswitch input through which commands can be routed to the computer through the Firewire interconnects.

All 1/O signal and commands to be EMI filtered as required.

General operation characteristics of the software interface include the following:
Supports 30 frames per second update rate at full resolution;
Single frame capture image storage;
Real-time capture;
Supports image stabilization;
Supports footswitch control.

The present camera system preferably provides single fault protection, and is compliant with the CE mark requirements and the safety requirements of IEC-60601-1, UL2601 and CSA.

It will be appreciated therefore, that a digital intraoral camera according to the present invention provides an improvement in the intraoral camera art, and is otherwise a useful and valuable contribution to the state of that art.

## Claims

1. An intraoral digital camera system comprising:
an intraoral digital camera (10) operatively connected to a docking station (11); said docking station (11) comprising:
control circuitry to convert a video signal from said camera (10) to a digital signal compatible with a digital output connection exiting from said docking station (11);
a light source having a lamp; and
a holster to support said camera (10), **characterised in that** said holster includes means to sense the presence of said camera (10) in said holster for automatic activation of said light source and said camera (10) when said camera is removed from said holster.

2. The intraoral digital camera system of claim 1, wherein said video signal from said camera (10) is S-Video.

3. The intraoral digital camera system of claim 1 or 2, wherein said digital output connection is compliant with the IEEE 1394 A standard.

4. The intraoral digital camera system of any one of claims 1 to 3, wherein said docking station is configured to be integrated into dental office furniture, and wherein said docking station (11) has a footprint equal to or smaller than about 150 mm x 150 mm x 50 mm.

5. The intraoral digital camera system of claim 4, wherein said dental office furniture is a dental chair.

6. The intraoral digital camera system of claim 4, wherein said docking system is adapted to be mounted under the delivery unit of a dental chair.

7. The intraoral digital camera system of any one of claims 1 to 6, wherein said light source comprises a thermostatic circuit breaker .

8. The intraoral digital camera system of claim 7, wherein said thermostatic circuit breaker is adapted to cut off the lamp current if the temperature of said light source assembly rises above a predetermined temperature level.

9. The intraoral digital camera system of any one of claims 1 to 8, wherein said means to sense the presence of said camera (10) in said holster is adapted to turn off the lamp and to blank the video signal when the camera is in the holster.

10. The intraoral digital camera system of any one of claims 1 to 9, wherein said means to sense the presence of said camera (10) in said holster comprises a magnetic sensing arrangement or a sensor assembly.

11. The intraoral digital camera system of claim 10, wherein said magnetic sensing arrangement comprises a magnet interacting with Hall effect or a magneto-resistive element.

12. The intraoral digital camera system of claim 10, wherein said sensor assembly comprises a LED/photo-transistor set.

13. The intraoral digital camera system of any one of claims 1 to 12, wherein said docking station (11) further includes a power supply board and a 12 VDC input connector.

14. The intraoral digital camera system of any one of claims 1 to 13, further comprising a power supply, said power supply having a connection to an AC input and a connection to said docking station.

15. The intraoral digital camera system of claim 13, wherein said docking station. (11) includes an input connector to receive signals from said intraoral digital camera (10); said power supply board has regulator and filter circuits; and said light source has a fan.

16. The intraoral digital camera system of any of claims 1 to 15, wherein said control circuitry is configured to convert S-Video signals from said camera (10) to IEEE 1394 A standard video signals for said digital output connection (12).

## Patentansprüche

1. Intraorales Digitalkamera-System, das umfasst:
eine intraorale Digitalkamera (10), die mit einer Aufnahmestation (11) operativ verbunden ist, wobei die Aufnahmestation (11) umfasst:
eine Regelschaltung zur Überführung eines Videosignals aus der Kamera (10) in ein digitales Signal, das mit einem digitalen Ausgabeanschluss, der aus der Aufnahmestation (11) führt, kompatibel ist;
ein Lichtquelle, die eine Lampe aufweist; und
einen Halfter zum Stützen der Kamera (10), **dadurch gekennzeichnet, dass** der Halfter Mittel umfasst, um die Gegenwart der Kamera (10) im Halfter zur automatischen Aktivierung der Lichtquelle und der Kamera (10), wenn die Kamera aus dem Halfter entfernt wird, festzustellen.

2. Intraorales Digitalkamera-System nach Anspruch 1, worin das Videosignal aus der Kamera (10) S-Video ist.

3. Intraorales Digitalkamera-System nach Anspruch 1 oder 2, worin der digitale Ausgabeanschluss dem IEEE 1394 A-Standard entspricht.

4. Intraorales Digitalkamera-System nach einem der Ansprüche 1 bis 3, worin die Aufnahmestation so konfiguriert ist, dass sie in einer Zahnarztpraxiseinrichtung integriert werden kann, und worin die Aufnahmestation (11) eine Aufstandsfläche aufweist, die gleich oder kleiner der von ca. 150 mm x 150 mm x 50 mm ist.

5. Intraorales Digitalkamera-System nach Anspruch 4, worin die Zahnarztpraxiseinrichtung ein Zahnbehandlungsstuhl ist.

6. Intraorales Digitalkamera-System nach Anspruch 4, worin das Aufnahmesystem so ausgestaltet ist, um unter das Zufiihrungsaggregat eines Zahnbehandlungsstuhls montiert zu werden.

7. Intraorales Digitalkamera-System nach einem der Ansprüche 1 bis 6, worin die Lichtquelle einen thermostatischen Trennschalter aufweist.

8. Intraorales Digitalkamera-System nach Anspruch 7, worin der thermostatische Trennschalter so ausgeführt ist, um den Lampenstrom abzuschalten, wenn die Temperatur der Lichtquellenanordnung über ein bestimmtes Temperaturniveau steigt.

9. Intraorales Digitalkamera-System nach einem der Ansprüche 1 bis 8, worin das Mittel zur Feststellung der Gegenwart der Kamera (10) im Halfter so ausgestaltet ist, um die Lampe auszuschalten und das Videosignal zu verhindern, wenn die Kamera im Halfter ist.

10. Intraorales Digitalkamera-System nach einem der Ansprüche 1 bis 9, worin das Mittel zur Feststellung der Gegenwart der Kamera (10) im Halfter eine magnetische Messanordnung oder eine Sensoranordnung aufweist.

11. Intraorales Digitalkamera-System nach Anspruch 10, worin die magnetische Messanordnung einen Magneten aufweist, der mit einem Hall-Effekt oder mit einem magnetoresistiven Element zusammenwirkt.

12. Intraorales Digitalkamera-System nach Anspruch 10, worin die Sensoranordnung eine LED/Phototransistor-Anordnung aufweist.

13. Intraorales Digitalkamera-System nach einem der Ansprüche 1 bis 12, worin die Aufnahmestation (11) ferner ein Stromzufuhrschaltpult und eine 12 VDC-Eingabeverbindung aufweist.

14. Intraorales Digitalkamera-System nach einem der Ansprüche 1 bis 13, das ferner eine Stromquelle aufweist, wobei die Stromquelle eine Verbindung zu einer AC-Eingabe und eine Verbindung zur Aufnahmestation aufweist.

15. Intraorales Digitalkamera-System nach Anspruch 13, worin die Aufnahmestation (11) eine Eingabeverbindung aufweist, um Signale aus der intraoralen Digitalkamera (10) zu empfangen, wobei das Stromzufuhrschaltpult Regel- und Filterschaltungen aufweist, und die Lichtquelle ein Gebläse aufweist.

16. Intraorales Digitalkamera-System nach einem der Ansprüche 1 bis 15, worin die Regelschaltung so konfiguriert ist, um S-Videosignale aus der Kamera (10) in IEEE 1394 A-Standard-Videosignale für den digitalen Ausgabeanschluss (12) zu überführen.

## Revendications

1. Système de caméra numérique intra-orale comprenant :
une caméra numérique intra-orale (10) en relation fonctionnelle avec une station de connexion (11) ; la station de connexion (11) comprenant :
un circuit de commande pour convertir un signal vidéo provenant de la caméra (10) en un signal numérique compatible avec une connexion de sortie numérique de la station de connexion (11) ;
une source lumineuse comportant une lampe ; et
un support pour supporter la caméra (10), ledit support ayant des moyens pour détecter la présence de la caméra (10) dans son support, en vue d'activer automatiquement la source lumineuse et la caméra (10) quand on enlève celle-ci de son support.

2. Système de caméra numérique intra-orale selon la revendication 1, dans lequel le signal vidéo provenant de la caméra (10) est un S-Vidéo.

3. Système de caméra numérique intra-orale selon la revendication 1 ou 2, dans lequel la connexion de sortie numérique est conforme à la norme IEEE 1394A.

4. Système de caméra numérique intra-orale selon l'une quelconque des revendications 1 à 3, dans lequel le support de connexion (11) est conçue pour être intégrée dans un mobilier de cabinet dentaire et présente un encombrement égal ou inférieur à environ 150 mm x 150 mm x 50 mm.

5. Système de caméra numérique intra-orale selon la revendication 4, dans lequel le mobilier de cabinet dentaire est constitué par un siège de dentiste.

6. Système de caméra numérique intra-orale selon la revendication 4, dans lequel le système de connexion est apte à être monté sous l'unité de distribution d'un siège de dentiste.

7. Système de caméra numérique intra-orale selon l'une quelconque des revendications 1 à 6, dans lequel la source lumineuse comprend un coupe-circuit thermostatique.

8. Système de caméra numérique intra-orale selon la revendication 7, dans lequel le coupe-circuit thermostatique est apte à couper le courant de la lampe si la température de l'ensemble de source lumineuse monte au-dessus d'un niveau de température prédéterminé.

9. Système de caméra numérique intra-orale selon l'une quelconque des revendications 1 à 8, dans lequel les moyens pour détecter la présence de la caméra (10) dans son support sont aptes à éteindre la lampe et à supprimer le signal vidéo quand la caméra se trouve dans le support.

10. Système de caméra numérique intra-orale selon l'une quelconque des revendications 1 à 9, dans lequel les moyens pour détecter le présence de la caméra (10) dans son support comprennent un dispositif de détection magnétique ou un ensemble de détection.

11. Système de caméra numérique intra-orale selon la revendication 10, dans lequel le dispositif de détection magnétique comprend un aimant en interaction avec l'effet Hall ou un élément magnétorésistant.

12. Système de caméra numérique infra-orale selon la revendication 10, dans lequel l'ensemble de détection comprend un ensemble diode lumineuse/phototransistor.

13. Système de caméra numérique intra-orale selon l'une quelconque des revendications 1 à 12, dans lequel la station de connexion (11) comprend également une carte d'alimentation et un connecteur d'entrée à 12 volts en courant continu.

14. Système de caméra numérique intra-orale selon l'une quelconque des revendications 1 à 13, comprenant également une alimentation, ladite alimentation ayant un branchement à une entrée à courant alternatif et un branchement à la station de connexion.

15. Système de caméra numérique intra-orale selon la revendication 13, dans lequel la station de connexion (11) comprend un connecteur d'entrée pour recevoir des signaux provenant de la caméra numérique intra-orale (10) ; la carte d'alimentation a des circuits de régulateur et de filtre ; et la source lumineuse a un ventilateur.

16. Système de caméra numérique intra-orale selon l'une quelconque des revendications 1 à 15, dans lequel le circuit de commande est conçu pour convertir les signaux S-Vidéo provenant de la caméra (10) en signaux vidéo IEEE 1394 A pour la connexion de sortie numérique (12).
